# EUROPEAN PATENT SPECIFICATION

(11) **EP 3 054 942 B1**
(45) Date of publication and mention of the grant of the patent: **24.03.2021**
(21) Application number: 14852168.5
(22) Date of filing: 07.10.2014
(51) Int. Cl.: A61K 31/335, A61P 27/02

(54) **TREATMENTS FOR PROLIFERATIVE VITREORETINOPATHY**
BEHANDLUNGEN FÜR PROLIFERATIVE VITREORETINOPATHIE
TRAITEMENTS DE VITRÉORÉTHINOPATHIE PROLIFÉRANTE

(30) Priority: 07.10.2013 US 201361887747 P
(43) Date of publication of application: 17.08.2016
(73) Proprietor: Mor Research Applications Ltd., 6971054 Tel Aviv (IL)
(72) Inventor: POLLACK, Ayala, 7635109 Rehovot (IL); DVASHI, Zeev, 5552504 Kiryat Ono (IL)
(74) Representative: Schulz Junghans Patentanwälte PartGmbB
(86) International application number: PCT/IL2014/050882
(87) International publication number: WO 2015/052714

(56) References cited:
- WO-A1-02/48135
- WO-A1-03/097615
- AARON GARDNER ET AL: "The Critical Role of TAK1 in Accentuated Epithelial to Mesenchymal Transition in Obliterative Bronchiolitis after Lung Transplantation", AMERICAN JOURNAL OF PATHOLOGY., vol. 180, no. 6, 1 June 2012 (2012-06-01), pages 2293-2308, XP055338500, US ISSN: 0002-9440, DOI: 10.1016/j.ajpath.2012.02.022
- STRIPPOLI, RAFFAELE ET AL.: 'Inhibition of transforming growth factor-activated kinase 1 (TAK1) blocks and reverses epithelial to mesenchymal transition of mesothelial cells.' PLOS ONE vol. 7, no. 2, 27 February 2012, page E31492, XP055331694 Retrieved from the Internet: <URL:http://citeseerx.ist.psu.edu/viewdoc/d ownload ? doi=10.1.1.272.1859&rep=rep1&type=pdf> [retrieved on 2012-02-27]
- TAKAHASHI, E. ET AL.: 'Tumor necrosis factor-alpha regulates transforming growth factor-beta- dependent epithelial-mesenchymal transition by promoting hyaluronan- CD 44-moesin interaction.' J BIOL CHEM vol. 285, no. 6, 04 December 2009, pages 4060 - 73., XP055331701 Retrieved from the Internet: <URL:http://www. jbc.org/content/285/6/4060.long> [retrieved on 2009-04-12]
- ZEEV DVASHI ET AL.: 'TGF-betal Induced Epithelial-Mesenchymal Transition in RPE cell is Mediated by TAK-1 Activation' INVEST OPHTHALMOL VIS SCI 2014; vol. 55, 04 May 2014, pages 9 - 10, XP008183229 Retrieved from the Internet: <URL:http://abstracts.iovs.org/cgi/content/ short/55/5/1093>
- P Broglie: "Transforming Growth Factor Beta-activated Kinase 1 (TAK1) Kinase Adaptor, TAK1-binding Protein 2, Plays Dual Roles in TAK1 Signaling by Recruiting Both an Activator and an Inhibitor of TAK1 Kinase in Tumor Necrosis Factor Signaling Pathway", THE JOURNAL OF BIOLOGICAL CHEMISTRY, vol. 285, no. 4, 2 December 2009 (2009-12-02), pages 2333-2339, XP55509471,
- Kazunori Inoue ET AL: "Maxacalcitol ameliorates tubulointerstitial fibrosis in obstructed kidneys by recruiting PPM1A/VDR complex to pSmad3", Laboratory Investigation, vol. 92, no. 12, 27 August 2012 (2012-08-27), pages 1686-1697, XP55509474, The United States and Canadian Academy of Pathology, Inc. ISSN: 0023-6837, DOI: 10.1038/labinvest.2012.107

## Description

### FIELD

Provided herein are methods for treatment for proliferative vitreoretinopathy (PVR) by inhibiting the activity of transforming growth factor β activated kinase 1 (TAK-1). Pharmaceutical compositions for use in the described treatments are also provided.

### BACKGROUND

Proliferative vitreoretinopathy (PVR) is a scarring process that develops as a complication during retinal detachment (RD) and it is the most common cause of surgical failure upon RD treatment (Ho et al., Br J Ophthalmol 1985, 69:584-587). PVR is a dynamic process characterized by the formation of fibrotic tissue on the detached retina, preventing the reattachment of the retina and finally may cause blindness (Lambert et al., Seminars in ophthalmology 1995, 10:49-52). Retinal pigment epithelial (RPE) cells, which are normally located in the external cell layer of the retina, are the most critical contributors to the development of fibrotic diseases of the eye. During PVR, RPE cells undergo transformation into fibroblast-like cells through a process known as the epithelial-mesenchymal transition (EMT) (Takahashi et al., J Biol Chem 2010, 285:4060-4073). In the process of converting from epithelial into mesenchymal cells, they lose their epithelial characteristics such as polarity and specialized cell-to-cell contact, and acquire migratory mesenchymal properties (Grisanti et al., Invest Ophthalmol Vis Sci 1995, 36:391-405). These processes are mediated by the expression of cell surface molecules, cytoskeletal reorganization, and extracellular matrix (ECM) components (Thiery et al., Cell 2009, 139:871-890; Kalluri et al., J Clin Invest 2009, 119:1420-1428). EMT can be triggered by different signaling molecules such as epidermal growth factor (EGF) and fibroblast growth factor (FGF), however transforming growth factor beta-1 (TGF-β1) is considered the main regulator of EMT (Garweg et al., Surv Ophthalmol 2013, 58:321-329; Lamouille et al., I 2007, 178:437-451; Charteris, Br J Ophthalmol 1995, 79:953-960).

TGF-β-mediated EMT has been observed in a variety of cell types, including lens epithelial cells, corneal epithelial cells and others (Saika S, Lab Invest 2006, 86:106-115). TGF- β is a multifunctional cytokine with an array of biological effects such as cell growth, differentiation, immunomodulation by two-edged sword effect, oxidative stress and Endoplasmic Reticulum (ER) stress (Desmouliere et al., J Cell Biol 1993, 122:103-111; Yoon et al., Oncogene 2005, 24:1895-1903). Intracellular signaling downstream to the TGF- β receptor complexes is mediated by the Smads family, the canonical pathway (Zhang et al., Cell Res 2009, 19:128-139). Recent reports have demonstrated that transforming growth factor β activated kinase 1 (TAK1), a member of the mitogen-activating protein (MAP) kinase kinase kinase family, is involved in the TGF-β signaling in the non-canonical pathway (Mu Y et al., Cell Tissue Res 2011, 347:11-20; Yamaguchi et al., Science 1995, 270:2008-2011; Kajino et al., J Biol Chem 2007, 282:9475-9481). But to date, the roles of TAK1 in RPE cell signaling and mediating PVR development were unknown. TAK1 is a serine/threonine kinase that is rapidly activated by TGF-β1 and subsequently activates other MAP kinases such as p38 (Ma et al., Am J Physiol Renal Physiol 2011, 300:F1410-1421; Wang et al., J Biol Chem 1997, 272:22771-22775). Moreover, studies indicate that TAK1 can regulate TGF-β-induced activation of Smad signaling by inducing Smad7 expression and also interfering with R-Smad transactivation by direct interaction with the MH2 domain of Smad proteins (Brown et al., J Cell Biochem 2007, 101:9-33). In addition to the role of TAK1 in the regulation of Smad function, there is cross-talk between the Smad and downstream targets of TAK1 such as p38 MAPK and ATF2 in the regulation of certain TGF-β1 target gene expression (Zhang et al., Cell Res 2009, 19:128-139; Mu Y et al., Cell Tissue Res 2011, 347:11-20). Even though TAK1 activation is associated with TGF-β1 signaling, it is well known that its activation can also be caused by various stimuli including: environmental stress, pro-inflammatory cytokines such as tumor necrosis factor-alpha (TNF-α), interleukin (IL)-1 and lipopolysaccharides (LPS) (Conner et al., Biochem J 2006, 399:427-434). Activated TAK1 can transduce signals to several downstream signaling cascades, including the MKK4/7-JNK, MKK3/6-p38 MAPK, and Nuclear Factor-kappa B (NF-kB)-inducing kinase (NIK)-IkB kinase (IKK) (Hanada et al., JBiol Chem 2001, 276:5753-5759).

Currently available surgical options for RD management are pneumatic retinopexy, scleral buckling, vitrectomy and pars plana vitrectomy (PPV). Although PVR is primarily managed surgically and there is large improvement in the technology, the numbers of PVR patients remain similar between the years of 1988 to 2003. Possible explanation to this high rate of PVR can be explained by the inability to prevent some level of cell adhesion and pathological changes to occur utilizing vitrectomy. Thus, there is a continuing need to develop therapies for PVR that will enable better treatment outcomes for RD.

### SUMMARY

Disclosed herein is the observation that TAK1 mediates TGF-β1-induced EMT in RPE cells. This discovery enables the development of pharmaceutical compositions for treatment of PVR, and methods of their use.

Accordingly, provided herein is a pharmaceutical composition for prevention or treatment of proliferative vitreoretinopathy (PVR) in a subject, which includes an inhibitor of at least one of the activation or kinase activity of transforming growth factor β activated kinase 1 (TAK-1), wherein the inhibitor is selected from 5Z-7-oxozeaenol or an activator of protein phosphatase 6 (PP6). Similarly formulated compositions are also described for inhibiting or preventing EMT in RPE cells

Also described herein are methods for prevention or treatment of proliferative vitreoretinopathy (PVR) in a subject, which include administering to a subject in need thereof, a therapeutically effective amount of an inhibitor of at least one of the activation or kinase activity of transforming growth factor β activated kinase 1 (TAK-1), thereby preventing or treating the PVR.

Additionally described are methods for inhibiting epithelial-mesenchymal transition (EMT) in retinal pigment epithelial cells in a subject, which include administering to a subject in need thereof, a therapeutically effective amount of an inhibitor of at least one of the activation or kinase activity of transforming growth factor β activated kinase 1 (TAK-1), thereby preventing or treating the PVR.

The foregoing and other objects, features, and advantages will become more apparent from the following detailed description, which proceeds with reference to the accompanying figures.

### BRIEF DESCRIPTION OF THE DRAWINGS

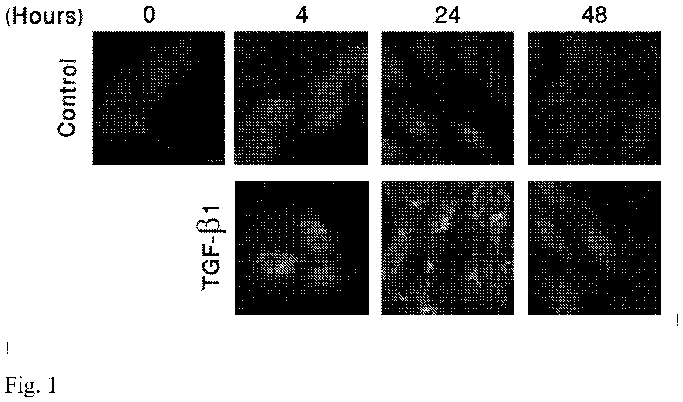

**Figure 1** shows that TAK1 is activated upon TGF- β1 stimulation in RPE cells. RPE cells were treated with TGF- β1 (2.5ng/ml) for the indicated times or left untreated. The cells were then immunostained with phospho-Thr 187 TAK1 antibodies (green) and DAPI (blue). Representative photographs of three independent experiments are shown. Scale bar is 10µm for all images.

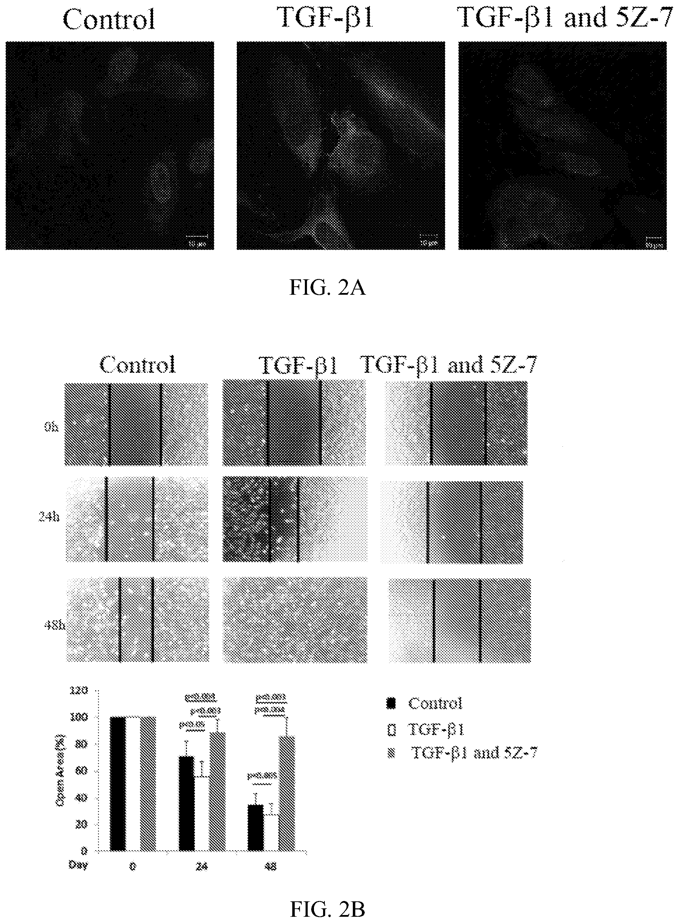

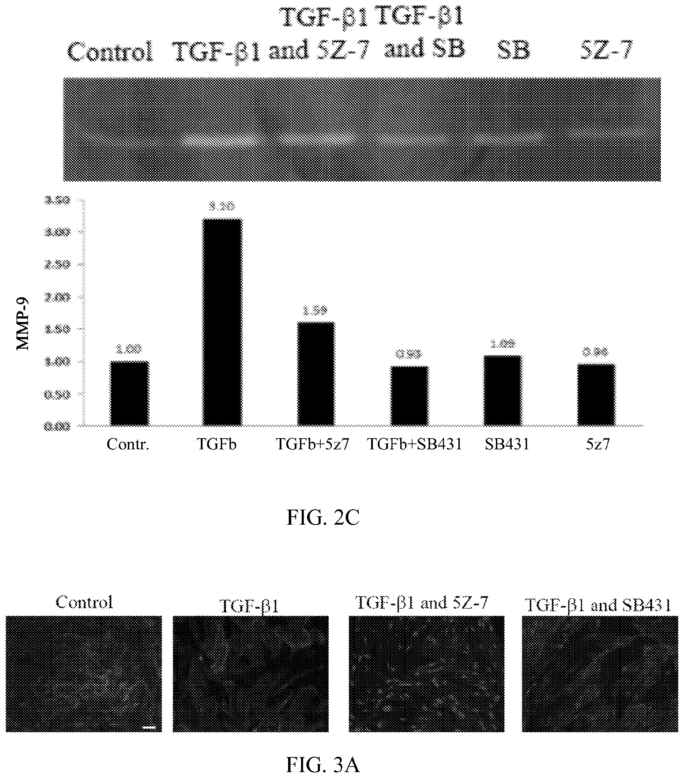

**Figure 2** shows that TAK1 regulates an EMT phenotype in RPE cells upon TGF-β stimulation. **Figure 2A****:** RPE cells were serum-starved for 16 hours, pretreated with 5Z-7-oxozeaenol (1µM) or left untreated for 1 hour. The cells were then treated with TGF-β1 (2.5ng/ml) for 24h and immunostained with α-SMA antibodies (red) and DAPI (blue). Representative photographs of three independent experiments. **Figure 2B****:** RPE cells were serum-starved for 16 hours, then treated with mitomycin C (10ng/ml) for 3h. Thereafter, the cells were pretreated with 5Z-7-oxozeaenol (1µM) or Dimethyl sulfoxide (DMSO) for 1 hour. Following this process a scratch was performed in the cell monolayer and the serum free medium was supplemented with TGF-β1 (2.5ng/ml) or left unsupplemented. Scratches were photo-documented at the indicated times (top panel) and their width was measured using Image-J software. The histogram (bottom panel) demonstrates the percentage of remaining gap at each time point, relative to initial gap width, based on three independent experiments. Bars are Mean±SD. **Figure 2C****:** RPE cells were serum-starved for 16 hours then pretreated with 5Z-7-oxozeaenol (1µM), SB431542 (10µM), or DMSO for 1 hour. The medium was then replaced with serum-free medium with or without TGF-β1 (2.5ng/ml) and supernatants from the different treatments were collected after 24 hours. Total MMP-9 activities were processed by gelatin zymography (top panel) and the band intensity values were calculated by Quantity One® 1-D analysis software. A histogram demonstrating secretion levels of MMP-9 in the different treatments is shown (bottom panel).

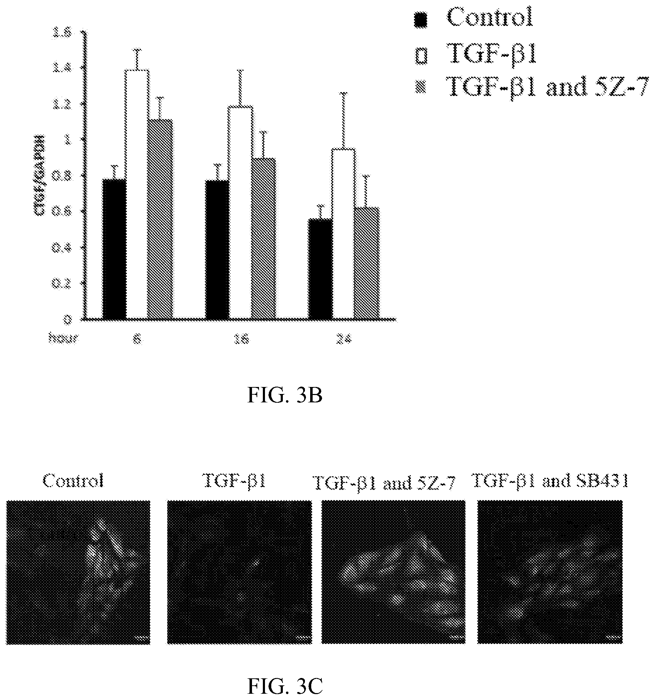

**Figure 3** shows that TGF- β regulates morphological and transcriptional changes in RPE cells through TAK1. **Figure 3A****:** RPE cells were plated on fibronectin-coated glass coverslips, serum-starved for 16 hours, pretreated with 5Z-7-oxozeaenol (1µM) or SB431542 (10µM), or DMSO for 1 hour, then treated with or without TGF- β 1 for 2 days. Following treatment, the cells were stained with rhodamine-phalloidin (actin fibers-green) and DAPI (blue) and visualized by confocal microscopy. The cells size is indicated by a white arrow. Scale bars: 20µM. Figure 3B: Serum-starved RPE cells pretreated with 5Z-7-oxozeaenol (1µM) or DMSO for 1 hour, and were exposed to TGF-β as in Figure 3A. Total RNA was extracted at each time point and qPCR was performed. Transcription levels of CTGF were determined after 6h, 16h and 24h. Bars represent the specific mRNA amount relative to GAPDH mRNA in the same samples. All experiments were performed in triplicates. A representative histogram from two independent experiments is shown. **Figure 3C****:** RPE cells were plated and treated as in **Figure 3A****.** Following treatment, the cells were immunostained with E-cadherin antibodies (green) and DAPI (blue). Representative photographs of two independent experiments are shown.

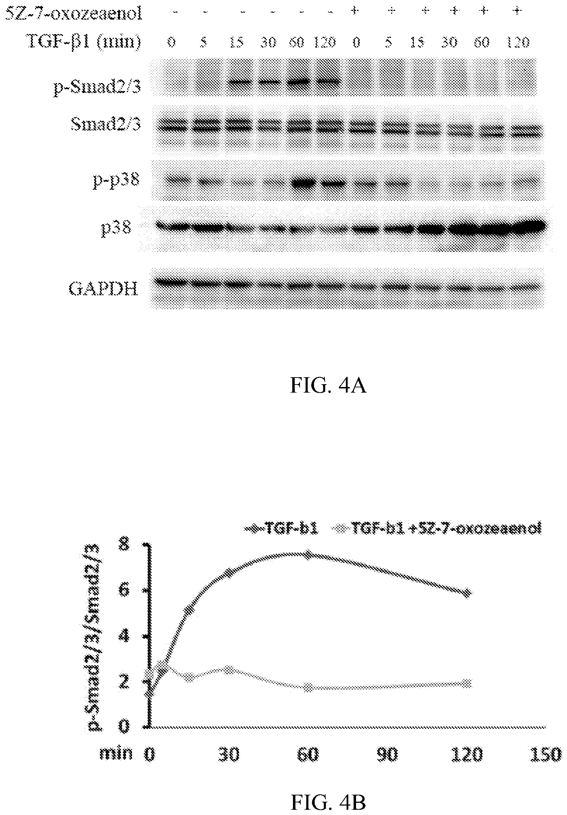

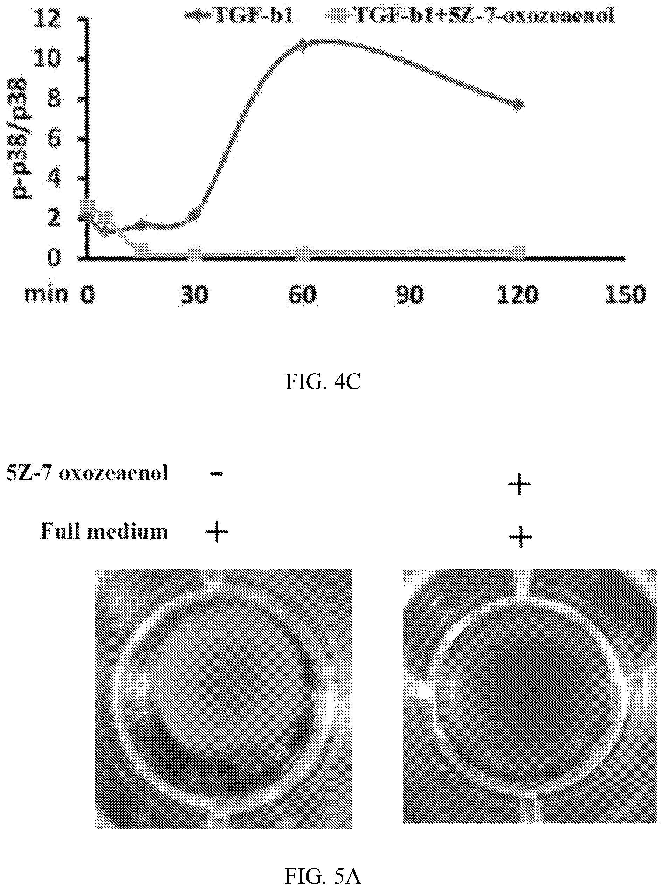

**Figure 4** shows that Inhibition of TAK1 abolishes the activation of TGF-β signaling cascades. **Figure 4A****:** Serum-starved RPE cells were pretreated with or without with 5Z-7-oxozeaenol (1µM) for 1 hour, and then with TGF-β (2.5ng/ml) for the indicated times. Total protein extracts were analyzed by western blot using the indicated antibodies. The blot shows a representative result of four independent experiments. **Figure 4B****:** Levels of phospho-Smad2/3 were quantified and normalized to total Smad2/3 **Figure 4C****:** Levels of phospho-p38 were quantified and normalized to total p38.

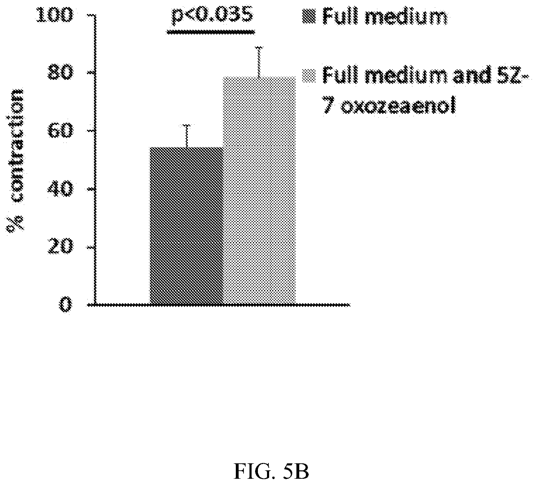

**Figure 5** shows that TAK1 is a general regulator of the EMT process in RPE cells. **Figure 5A****:** RPE cells were pre-treated with or without 5Z-7-oxozeaenol (1µM) and seeded in collagen lattices in full medium. The experiments were performed in triplicates. Lattices were photo-documented after 24 hours and measured using Image-J software. **Figure 5B****:** The histogram demonstrates the percentage of lattice area relative to initial gel area, based on three independent experiments. Bars are Mean±SD.

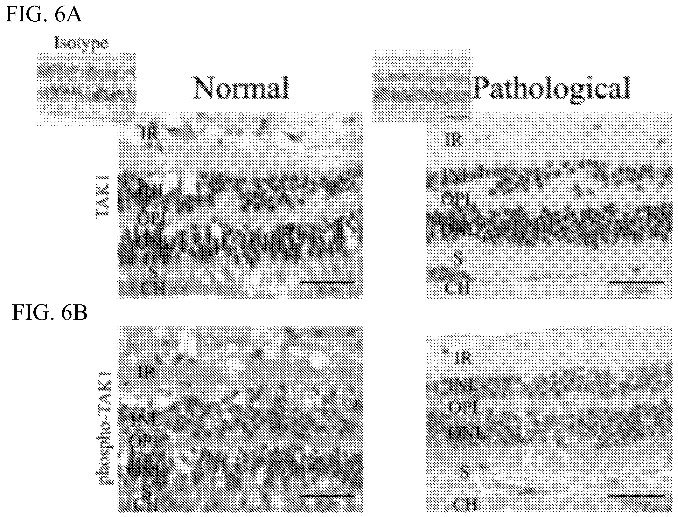

**Figure 6** shows aberrant Activity of TAK1 in Human Pathologic Retina. **Figure 6A****:** Retinal specimens were embedded in paraffin and immunostained with anti-TAK1 antibodies and hematoxylin. **Figure 6B****:** Retinal specimens were treated as in Figure 6A and stained with phospho-Thr 187 TAK antibodies and hematoxylin. This demonstrates aberrant activity of TAK1 in the retina of blind painful eye. IR -inner retinal layers; INL- inner nuclear layer; OPL-outer plexiform layer; ONL- outer nuclear layer; S- photoreceptor segment; SC- sclera (Scale bar = 50µm). The causes for pathology (blind painful eye) in these cases were diverse, including trauma, end stage glaucoma, or retinal detachment after unsuccessful surgeries. The specimens with presumed normal retina were obtained from enucleated eyes for intraocular malignancy - choroidal melanoma.

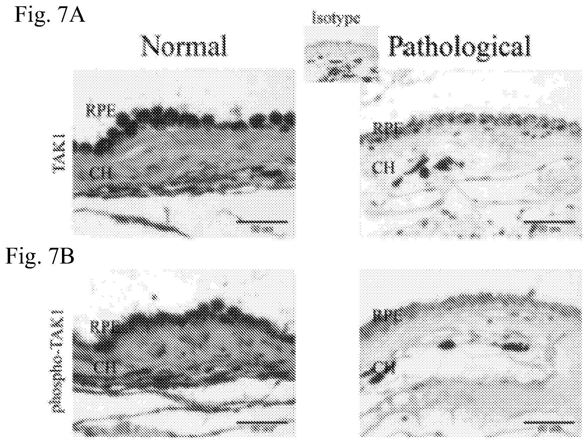

**Figure 7** shows attenuated TAK1 Activity in RPE Cells with pathology. **Figure 7A****:** RPE of a retinal specimen was embedded in paraffin, and immunostained with TAK1 antibodies and hematoxylin. (N=5). **Figure 7B****:** Retinal specimen treated as in Figure 7A and stained with phospho-Thr 187 TAK antibodies and hematoxylin, This demonstrating aberrant activity of TAK1 in the RPE cells of blind painful eye (Scale bar = 50µm). As in Figure 6, the causes for pathology (blind painful eye) in these cases were diverse, including trauma, end stage glaucoma, or retinal detachment after unsuccessful surgeries. The specimens with presumed normal retina were obtained from enucleated eyes for intraocular malignancy - choroidal melanoma.

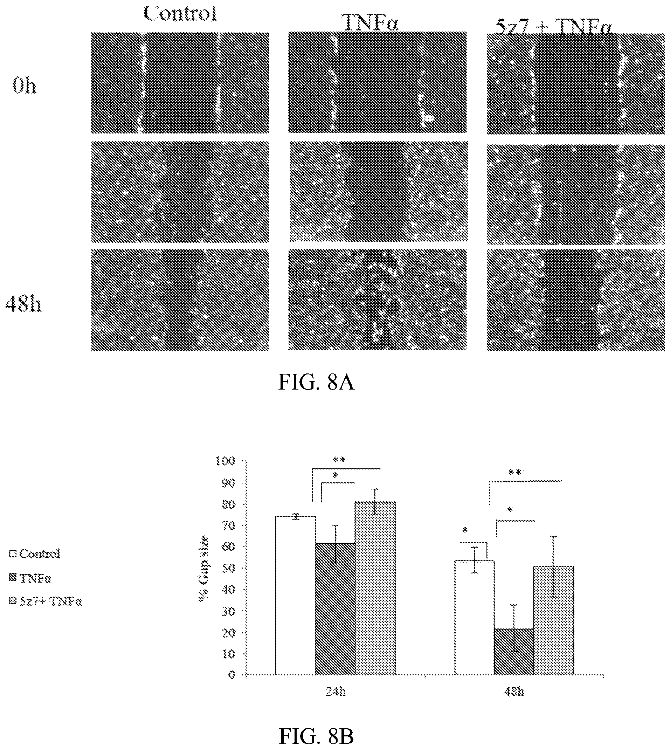

**Figure** 8 shows that a TAK1 inhibitor reduced the migration rates of RPE cells treated with TNFα. **Figure 8A****:** Cell migration of RPE cells in *in vitro* scratch wound healing assay. RPE cells were treated with Mitomycin C for 3 hours. After the cells were treated with 1µM 5Z-7 for 1 hour before adding 20ng/ml TNFα and wounding on day 0. Photographs were taken at day 1 and 2, respectively, after the wound was made. **Figure 8B****:** Percentage of the gap size (*, *P* < 0.05, *t* test); (**, *P* < 0.01, *t* test).

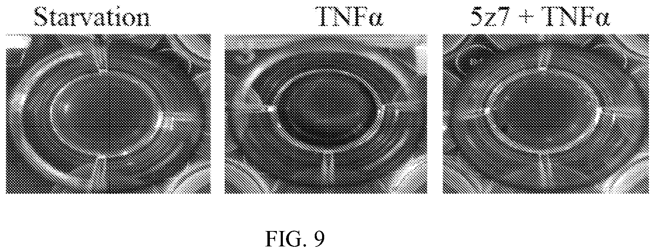

**Figure 9** shows TAK1 inhibitor reduced the contraction of collagen caused by TNFα in RPE cells. RPE cells were seeded in collagen lattices in serum starvation medium. The cells were pretreated with 1µM 5Z-7 or 1 hour before adding 50ng/ml TNFα. Lattice were photo-documented after 72 hours and measured using ImajeJ software.

### DETAILED DESCRIPTION

### I. Abbreviations

- **EMT**: epithelial-mesenchymal transition
- **PVR**: proliferative vitreoretinopathy
- **RD**: retinal detachment
- **RPE**: retinal pigment epithelial
- **TAK1**: transforming growth factor β activated kinase 1
- **TGF-β1**: transforming growth factor beta-1
- **TNFα**: tumor necrosis factor α

### II. Terms

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described below. The term "comprises" means "includes." The abbreviation, *"e.g."* is derived from the Latin *exempli gratia,* and is used herein to indicate a non-limiting example. Thus, the abbreviation *"e.g."* is synonymous with the term "for example."

In case of conflict, the present specification, including explanations of terms, will control. In addition, all the materials, methods, and examples are illustrative and not intended to be limiting.

**Abnormal:** Deviation from normal characteristics. Normal characteristics can be found in a control, a standard for a population, etc. For instance, where the abnormal condition is a disease condition, such as PVR, a few appropriate sources of normal characteristics might include an individual who is not suffering from the disease (*e.g.,* PVR), a condition associated with the disease (*e.g.* RD), or a population standard of individuals believed not to be suffering from the disease.

Likewise, abnormal may refer to a process that is associated with a disease, such as abnormal EMT in RPE cells. The term "associated with" includes an increased risk of developing the disease as well as the disease itself. For instance, a certain abnormality (such as an abnormality in TAK1 activation) can be described as being associated with the biological conditions of abnormal EMT and the development of PVR.

**Administration:** The introduction of a composition into a subject by a chosen route. Administration of an active compound or composition can be by any route known to one of skill in the art to be useful for a particular target area. Administration can be local or systemic. In examples of treating PVR, administration includes those routes by which an active compound can reach the intra-ocular space. Particular non-limiting examples include intra-ocular injection, topical drops, and intra-ocular implants.

**Analog, derivative or mimetic:** An analog is a molecule that differs in chemical structure from a parent compound, for example a homolog (differing by an increment in the chemical structure, such as a difference in the length of an alkyl chain), a molecular fragment, a structure that differs by one or more functional groups, a change in ionization. Structural analogs are often found using quantitative structure activity relationships (QSAR), with techniques such as those disclosed in Remington (The Science and Practice of Pharmacology, 19th Edition (1995), chapter 28). A derivative is a biologically active molecule derived from the base structure. A mimetic is a molecule that mimics the activity of another molecule, such as a biologically active molecule. Biologically active molecules can include chemical structures that mimic the biological activities of a compound. It is acknowledged that these terms may overlap in some circumstances.

**Effective amount of a compound:** A quantity of compound sufficient to achieve a desired effect in a subject being treated. An effective amount of a compound can be administered in a single dose, or in several doses, for example daily, during a course of treatment. However, the effective amount of the compound will be dependent on the compound applied, the subject being treated, the severity and type of the affliction, and the manner of administration of the compound.

**Inhibiting protein activity:** To decrease, limit, or block an action, function or expression of a protein. The phrase inhibit protein activity is not intended to be an absolute term. Instead, the phrase is intended to convey a wide-range of inhibitory effects that various agents may have on the normal (for example, uninhibited or control) protein activity. Inhibition of protein activity may, but need not, result in an increase in the level or activity of an indicator of the protein's activity. By way of example, this can happen when the protein of interest is acting as an inhibitor or suppressor of a downstream indicator. Thus, protein activity may be inhibited when the level or activity of any direct or indirect indicator of the protein's activity is changed (for example, increased or decreased) by at least 10%, at least 20%, at least 30%, at least 50%, at least 80%, at least 100% or at least 250% or more as compared to control measurements of the same indicator. Inhibition of protein activity may also be effected, for example, by inhibiting expression of the gene encoding the protein or by decreasing the half-life of the mRNA encoding the protein.

**Pharmaceutically acceptable carriers:** The pharmaceutically acceptable carriers useful in this disclosure are conventional. Remington's Pharmaceutical Sciences, by E. W. Martin, Mack Publishing Co., Easton, PA, 15th Edition (1975), describes compositions and formulations suitable for pharmaceutical delivery of the compounds herein disclosed.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

**Preventing or treating a disease:** Preventing a disease refers to inhibiting the full development of a disease, for example inhibiting the development of PVR in a person who has a detached retina or inhibiting the progression of EMT in RPE cells exposed to blood-borne cytokines. Treatment refers to a therapeutic intervention that ameliorates a sign or symptom of a disease or pathological condition after it has begun to develop. In particular examples, treatment of a disease can include inhibition of disease progression. For example, treatment of PVR can result from inhibition of EMT in RPE cells.

**Transforming growth factor-beta activated kinase 1 (TAK1):** TAK1 is a member of the MAPKKK family, and was first reported as a regulator of MAP kinase signaling induced by TGF-β or oxidative stress. TAK1 is known to be activated by stress signals as well as proinflammatory cytokines, and is involved in activation of p38 and JNK signaling. TAK1 was originally known as MAP3K7.

### III. Overview of Several Embodiments

Provided herein is a pharmaceutical composition for prevention or treatment of proliferative vitreoretinopathy (PVR) in a subject, which includes an inhibitor of at least one of the activation or kinase activity of transforming growth factor β activated kinase 1 (TAK-1).

In particular embodiments, the pharmaceutical composition includes a TAK1 inhibitor that inhibits the activation of TAK-1. In other embodiments, the TAK1 inhibitor inhibits the kinase activity of active TAK-1.

In a particular embodiment, theTAK1 inhibitor is 5Z-7-oxozeaenol.

In a further embodiment, the pharmaceutical composition includes an activator of protein phosphatase 6 (PP6).

Methods of using the provided pharmaceutical compositions for prevention or treatment of proliferative vitreoretinopathy (PVR) in a subject are also disclosed herein, which include administering a therapeutically effective amount of at least one of the described pharmaceutical compositions to a subject in need thereof, thereby preventing or treating the PVR.

In particular embodiments, the inhibitor is provided for administration during or after surgery for treatment of retinal detachment.

The pharmaceutical compositions for prevention or treatment of PVR can also be used for preventing or inhibiting epithelial-mesenchymal transition (EMT) in retinal pigment epithelial cells, and in methods for preventing or inhibiting epithelial-mesenchymal transition (EMT) in retinal pigment epithelial cells.

### IV. TAK1 Inhibition for Treatment of PVR

EMT in RPE is the root cause of PVR following retinal detachment. Prior to this disclosure, although TGF-β1 was associated with EMT in a variety of cell types, no direct association with EMT in retina had ever been determined. Moreover, the expression of TAK1 in retina, and the role of TGF-β1-activated TAK1 in retinal EMT, and by extension development of PVR was unknown.

Provided herein is the discovery that TAK1 is an essential mediating factor for TGF-β1-induced PVR following retinal detachment. This discovery enables the development of pharmaceutical agents that can be used to inhibit retinal EMT and development of PVR following retinal detachment.

The pharmaceutical compositions described herein include any agent that can inhibit the activation of TAK1, or can prevent the ability for activated TAK1 to phosphorylate its target molecules. Non-limiting examples of TAK1 inhibitory agents include small molecule inhibitors, peptides, antibodies, and antisense nucleic acids.

In particular embodiments, the agents for use in the described compositions and methods can inhibit the activation (e.g. phosphorylation) of TAK1, for example at the TAK1 Thr-187 residue. In particular embodiments, the agent is a blocking or neutralizing antibody that specifically recognizes TAK1 and prevents the phosphorylation of Thr-187.

In other embodiments, the agents for use in the described compositions and methods are zearalenones having hydroxyl groups at both of the 8- and 9-positions, for example 5Z-7 oxozeanol, and derivatives thereof. Description of TAK1-inhibitory zearalenone derivatives can be found at least in International Patent Publication No. WO2002/48135.

Certain phosphatases, including but not limited to protein phosphatase 1A (PPM1A), protein phosphatase 1B (PPM1B), protein phosphatase 6 (PP6), and dual specificityprotein phosphatase 14 (DUS14) are associated with maintenance of TAK1 in a non-activated (e.g. naive) state. In certain embodiments, increased activity of such phosphatases can inhibit activation of TAK1. In particular examples, the described composition includes the small molecule activator NPLC0393 or a derivative thereof, which can increase the activity of the PPM1A and/or PPM1B phosphatases (Wang et al., PLoS One 2010 Dec 6;5(12):e14230).

The ability for any of the described agents to inhibit the activation and/or activity of TAK1 can be determined (if not known) by any method known in the art, including those described in the Examples section below.

### V. Methods for Inhibition of EMT in the Retina and Treatment of PVR

The described TAK1-inhibiting compositions can be used in methods of inhibition of EMT in RPE cells and for treatment or prevention of PVR. EMT occurs in RPE cells following retinal trauma, such as retinal detachment. Accordingly, the methods of treatment described herein relate to administration of a TAK1 inhibiting agent to a subject who has experienced retinal trauma.

Surgery is the most common treatment for retinal trauma, and it is the scarring resultant from PVR which is known to decrease positive outcomes of RD surgery. In particular embodiments, the TAK1 inhibitor (such as 5Z-7 oxozeanol, and derivatives thereof) is administered to a subject following RD, but prior to RD surgery. In other embodiments, the TAK1 inhibitor is administered during surgery, or as a post-operative treatment. It will be appreciated that when administered post-operatively, the TAK1 inhibitor can be administered directly after RD surgery as well as throughout any recovery period following retinal trauma and RD surgery. In particular embodiments, the TAK1 inhibitor is administered within the first post-operative day, one, two, three, four, five, six days, or one, two, three, four, or more weeks after surgery.

As described below, the administration of a TAK1 inhibitor can be by any route and in any form suitable for delivery of the inhibitor to RPE cells.

### VI. Pharmaceutical Compositions and Modes of Administration

It is contemplated that the pharmaceutical agents for use in the described treatments can be supplied in any pharmaceutically acceptable compositions.

Among the pharmaceutical compositions specifically contemplated in the present disclosure are pharmaceutically acceptable acid or base addition salts of small molecule inhibitors of TAK1 (such as 5Z-7 oxozeanol, and derivatives thereof). The phrase "pharmaceutically acceptable acid or base addition salts" includes therapeutically active non-toxic acid and non-toxic base addition salt forms which a TAK1 inhibitor is able to form. Such compounds which have basic properties can be converted in their pharmaceutically acceptable acid addition salts by treating said base form with an appropriate acid. Appropriate acids comprise, for example, inorganic acids such as hydrohalic acids, *e.g.* hydrochloric or hydrobromic acid; sulfuric; nitric; phosphoric and the like acids; or organic acids such as, for example, acetic, propanoic, hydroxyacetic, lactic, pyruvic, oxalic, malonic, succinic (*i.e.* butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids.

Small molecule TAK1 inhibitors which have acidic properties may be converted in their pharmaceutically acceptable base addition salts by treating said acid form with a suitable organic or inorganic base. Appropriate base salt forms comprise, for example, the ammonium salts, the alkali and earth alkaline metal salts, *e.g.* the lithium, sodium, potassium, magnesium, calcium salts and the like, salts with organic bases, *e.g.* the benzathine, N-methyl-D-glucamine, hydrabamine salts, and salts with amino acids such as, for example, arginine, lysine and the like.

The terms acid or base addition salt also comprise the hydrates and the solvent addition forms of the small molecules in the described compositions. Examples of such forms are, for instance, hydrates, alcoholates and the like.

Also contemplated for use in methods and compositions described herein are sterochemcially isomeric forms of the described small molecule. The term stereochemically isomeric form includes all possible compounds made up of the same atoms bonded by the same sequence of bonds, but having different three-dimensional structures that are not interchangeable. Unless otherwise mentioned or indicated, the chemical designation of a compound encompasses the mixture of all possible stereochemically isomeric forms that the compound may possess. Such mixture may contain all diastereomers and/or enantiomers of the basic molecular structure of the compound. Also contemplated are all stereochemically isomeric forms in pure form or in admixture with each other.

Various intraocular delivery systems are known and can be used to administer the peptides, antibodies, and small molecules described herein. Such systems include, for example, encapsulation in liposomes, microparticles, microcapsules, recombinant cells capable of expressing therapeutic molecule(s) (see, *e.g.,* Wu et al., J. Biol. Chem. 262, 4429, 1987), construction of a therapeutic nucleic acid (expressing the described peptide or antibody) as part of a retroviral or other vector, and the like. Methods of introduction include, but are not limited to, intraocular injection, topical administrations, and as delivered by use of an intraocular implant. It is also contemplated that the described TAK1 inhibitors may be administered together with other biologically active agents.

In a specific embodiment, it may be desirable to administer the described pharmaceutical treatments implant (*e.g.,* implants formed from porous, non-porous, or gelatinous materials, including membranes, such as sialastic membranes or fibers), and the like. In another embodiment, therapeutic agents are delivered in a vesicle, in particular liposomes (see, *e.g.,* Langer, Science 249, 1527, 1990; Treat et al., in Liposomes in the Therapy of Infectious Disease and Cancer, Lopez-Berestein and Fidler (eds.), Liss, N.Y., pp. 353-365, 1989).

In yet another embodiment, the TAK1 inhibitor can be delivered in a controlled release system. In one embodiment, polymeric materials can be used (see, *e.g.,* Ranger et al., Macromol. Sci. Rev. Macromol. Chem. 23, 61, 1983; Levy et al., Science 228, 190, 1985; During et al., Ann. Neurol. 25, 351, 1989; Howard et al., J. Neurosurg. 71, 105, 1989). Other controlled release systems, such as those discussed in the review by Langer (Science 249, 1527 1990), can also be used.

The vehicle in which the TAK1 inhibitor is delivered can include pharmaceutically acceptable compositions of the compounds, using methods well known to those with skill in the art. For instance, in some embodiments, the agents described herein are typically contained in a pharmaceutically acceptable carrier. The term "pharmaceutically acceptable" means approved by a regulatory agency of the federal or a state government or listed in the U.S. Pharmacopoeia or other generally recognized pharmacopoeia for use in animals, and, more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable, or synthetic origin, such as peanut oil, soybean oil, mineral oil, sesame oil, and the like. The medium may also contain conventional pharmaceutical adjunct materials such as, for example, pharmaceutically acceptable salts to adjust the osmotic pressure, lipid carriers such as cyclodextrins, proteins such as serum albumin, hydrophilic agents such as methyl cellulose, detergents, buffers, preservatives and the like.

Examples of pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol, and the like. The therapeutic, if desired, can also contain minor amounts of wetting or emulsifying agents, or pH buffering agents. The therapeutics can take the form of solutions, suspensions, emulsion, tablets, pills, capsules, powders, sustained-release formulations, and the like. The therapeutic can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, and the like. A more complete explanation of parenteral pharmaceutical carriers can be found in Remington: The Science and Practice of Pharmacy (19th Edition, 1995) in chapter 95.

Embodiments of other pharmaceutical compositions are prepared with conventional pharmaceutically acceptable counter-ions, as would be known to those of skill in the art.

Therapeutic preparations will contain a therapeutically effective amount of at least one active ingredient, preferably in purified form, together with a suitable amount of carrier so as to provide proper administration to the patient. The formulation should suit the mode of administration.

The effective amount of a TAK1 inhibitor in the described pharmaceutical compositions can be determined by standard clinical techniques. The precise dose to be employed in the formulation will also depend on the route of administration, and should be decided according to the judgment of the health care practitioner and each patient's circumstances. Exemplary dosages of the individual compounds are described herein, but myriad other dosage regimens are encompassed by this disclosure. An example of an additional dosage range is 0.1 to 200 mg/kg body weight in single or divided doses. Another example of a dosage range is 1.0 to 100 mg/kg body weight in single or divided doses.

The specific dose level and frequency of dosage for any particular subject may be varied and will depend upon a variety of factors, including the activity of the specific compound, the metabolic stability and length of action of that compound, the age, body weight, general health, sex, diet, mode and time of administration, rate of excretion, drug combination, and severity of the condition of the host undergoing therapy.

The following examples are provided to illustrate certain particular features and/or embodiments. These examples should not be construed to limit the disclosure to the particular features or embodiments described.

### EXAMPLES

### Example 1: TGF-β1 Activates TAK1 MAP Kinase in RPE Cells

TAK1 is a known mediator of TGF-β1 signaling, but has not been detected (in either inactive or active forms) in RPE cells. This example shows that TAK1 is expressed and is activated in RPE cells.

To investigate the kinetics and the expression pattern of activated TAK1 in RPE cells upon TGF-β1 treatment, RPE cells were immunostained using phospho-TAK1 antibodies, which specifically recognize the activated form of TAK1 phosphorylated on Thr 187. As shown in Figure 1, TAK1 activation, observable in the non-nuclear staining, gradually increased in the treated cells, reaching a peak 24 hours post treatment and then declined. In contract, the control cells displayed only marginal levels of activated TAK1 with no significant changes, thus demonstrating the specific activation of TAK1 by TGF- β1 in the RPE cells.

The aberrant expression and activity of TAK1 in pathological retina and RPE cells is also shown in Figure 6A, Figure 6B (retina), Figure 7A, and Figure 7B (RPE cells).

### Example 2: Inhibition of TAK1 Halts the EMT Process upon TGF- β1 Stimulation in RPE cells

It is known that EMT can be mediated by a variety of factors. This example shows that TGF-β1-stimulated EMT occurring in RPE is mediated by activated TAK1.

EMT of RPE cells is known to be mediated by TGF-β 1 and characterized by increased expression of α-SMA, enhanced cell migration, and secretion of MMPs^{4,22}. To address a possible involvement of TAK1 in TGF-β1-induced RPE cell transdifferentiation, we studied the effects of specific TAK1 inhibitors on this process. A crucial step in transdifferentiation of RPE cells to myofibroblasts is the de novo synthesis of α-SMA(Hinz et al., Am J Pathol 2001, 159:1009-1020). To observe this process *in vitro,* we stimulated RPE cells with TGF- β1 for 2 days under serum-free conditions with or without the presence of a TAK1 inhibitor (5Z-7-oxozeaenol), or DMSO as a control (Figure 2A). As can be seen in figure 2A, while TGF- β1 considerably stimulated α-SMA expression, the TAK1 inhibitor significantly attenuated this process.

To further support the finding that TAK1 mediated EMT in RPE, we examined the migratory capacity of RPE cells, a well-known marker for EMT (Lee et al., J Cell Biol 2006, 172:973-981). Cell migration was tested by the scratch assay. The assay was performed on RPE cells pretreated or untreated with TAK1 inhibitor prior to stimulation with TGF-β1. As expected, addition of TGF- β1 significantly enhanced the migration of the RPE cells (Figure 2B, TGF-β1) compared to control cells. At 24 hours, in the TGF- β1 treated culture, a major portion of the cells had already migrated into the wounded area, displaying almost complete wound closure at 48 hours. In contrast, the addition of 5Z-7-oxozeaenol to TGF- β1 stimulated cells abolished their migratory capacity.

Emerging evidence indicates that matrix metalloproteinases (MMPs) can stimulate processes associated with EMT such as enhancement of cell invasion and migration (Lee et al., Invest Ophthalmol Vis Sci 2007, 48:4291-4299). Most reports suggest that predominance of MMP-2, -3 and -9 proteins correlate with worse prognosis. Examining the role of TAK1 in the activation of MMP-9 by gelatin zymography demonstrated that after 24 hours of exposure, TGF-β1 stimulated 92-kDa MMP-9 protein activity in RPE cells (Figure 2C). The activity of MMP-9 was significantly higher (3.2 fold) than the control group. Treatment of the cells with TAK1 inhibitor prior to TGF-β1 stimulation resulted in decreased MMP-9 activity, only 1.59 fold higher than the control group. Moreover, pre-treatment with SB431542, a specific inhibitor of the TGF-β1 receptor, completely abolished the activation of MMP-9. Thus, this result demonstrates that TGF-β1 is an inducer of MMP-9 activation in RPE cells and this phenomenon is mediated by TAK1.

In addition to the secretion of MMPs and increased migratory capacity, several morphological changes underlie EMT (Lamouille *et al*., 12007, 178:437-451). One of the phenotypes characterizing EMT is an increase in cell size mediated by TGF-β1 (Lamouille et al., I 2007, 178:437-451; Casaroli-Marano et al., Invest Ophthalmol Vis Sci 1999, 40:2062-2072). Examination of RPE cells treated with TGF-β1 confirmed our expectations: cells were indeed enlarged 24 hour post treatment (Figure 3A). In contrast, RPE cells that were treated with TAK1 inhibitor prior to TGF-β1 treatment demonstrated a minimal number of hypertrophic RPE cells. Concomitantly, we found that employing SB431542, a specific inhibitor of TGF-β1 receptor, abolished the increase in cell size, similarly to the results with TAK1 inhibitor (Figure 3A).

CTGF, an important stimulant of fibrosis, functions as a downstream mediator of TGF-β, stimulating cell proliferation and cell matrix deposition (He et al., Invest Ophthalmol Vis Sci 2008, 49:4078-4088). We examined the association between TGF-β1 and CTFG expression in RPE cells by Real Time PCR (figure 3B). Our results show that CTGF expression is significantly enhanced as early as 6 hours post TGF-β1 stimulation, and the effect lasts for at least 24 hours. Addition of the TAK1 inhibitor gradually reduced the levels of CTGF up to 24 hours post treatment, indicating that CTGF is a downstream mediator of TGF-β via TAK1 pathway.

A recent publication has shown that down-regulation of the cell-cell adhesion protein E-cadherin, was considered a hallmark of EMT in RPE cells (Tamiya et al., Invest Ophthalmol Vis Sci 2010, 51:2755-2763). To investigate the participation of TAK1 in the regulation of E-cadherin, we again treated RPE cells with 5Z-7-oxozeaenol or SB431542 prior to TGF-β 1 stimulation and immunostained the cells with anti-E-cadherin antibody. The result demonstrates that TAK1 is indeed involved in the regulation of E-cadherin expression (Figure 3C). Control RPE cells express high levels of E-cadherin that almost completely disappear upon TGF-β1 addition. Inhibition of TAK1 reverses the effect of TGF-β1, allowing normal E-cadherin expression and thus maintaining the naive form of the RPE cells as in the control (Figure 3C).

### Example 3: TAK1 Regulates TGF-β Signaling in RPE Cells

The results shown in Example 2 indicated that inhibition of TAK1 reduced the expression of α-SMA and cell migration. These processes are known to be regulated in RPE cells by the TGF-β1 signaling through Smad2/3 (Mu Y et al., Cell Tissue Res 2011, 347:11-20). This example explores whether TAK1 inhibition would have an effect on the phosphorylation of Smad2/3.

RPE cells were treated with or without 5Z-7-oxozeaenol followed by TGF-β1 treatment and Smad2/3 phosphorylation patterns were examined by immuno-blots. We observed that in TGF-β1 treated cells, phospho-Smad2/3 immediately increased and reached maximal activation at 60 minutes (Figure 4A). In contrast, in the cells that were pre-treated with 5Z-7-oxozeaenol, Smad2/3 activation was barely detectable. Calculating the ratio of phospho-Smad2/3 to total Smad2/3 at each time point clearly demonstrated that inhibition of TAK1 activity abolished Smad2/3 activation in TGF-β1-stimulated RPE cells (Figure 4B). Examination of the phosphorylation of p38, belonging to the non-canonical pathway of TGF-β1, revealed similar results to those obtained in the Smad2/3 signaling. Upon TGF-β1 stimulation, the phosphorylation of p38 increased, reaching a peak at 60 min (Figure 4A and C). In contrast, applying TAK1 inhibitor abolished p38 activation upon TGF-β1 stimulation (Figure 4C). These results demonstrate that inhibition of TAK1 halts both the canonical and non-canonical cascades of TGF-β 1.

### Example 4: TAK1 Activity is Essential for EMT of the RPE Cells

The transition of RPE cells to myofibroblasts is characterized, among other aspects, by increased contractile activity (Umazume et al., Invest Ophthalmol Vis Sci 2013, 54:1150-1159; Sakamoto et al., Curr Eye Res 1994, 13:451-459; West-Mays et al., Int J Biochem Cell Biol 2006, 38:1625-1631; Ma et al., Clinical & experimental ophthalmology 2012, 40:e76-86). To examine whether TAK1 is a general regulator of the EMT process affecting cell contractility, floating collagen matrix contraction assay was performed. The test was carried out in full serum with or without TAK1 inhibitor (Figure 5). While in the control cells significant contraction was observed after 24 hours, reaching 50% of the initial size, TAK1 inhibition impaired the capacity of the cells to contract. The collagen lattice remained nearly 80% from the initial size (Figure 5A and B).

### Example 5: 5Z-7-oxozeaenol for Treatment of PVR

The above examples demonstrate that the TAK1 inhibitor 5Z-7-oxozeaenol can inhibit the causative physiological pathways of PVR. Accordingly TAK1 inhibitors, such as 5Z-7-oxozeaenol, and derivatives thereof, can be used as pharmaceutical agents to treat subjects with retinal trauma (such as RD), who are at risk of developing PVR.

A subject with RD is treated upon diagnosis with a therapeutically effective amount of a pharmaceutical composition that includes 5Z-7-oxozeaenol, or a derivative thereof, as the active ingredient. Such subjects can be treated with eye drops to administer the pharmaceutical composition either before and/or after surgery for treatment of the RD. Success of the treatment can be measured by inhibition or prevention of the fibrosis and scarring associated with PVR, and by improved outcomes of RD-treatment surgery.

In view of the many possible embodiments to which the principles of the disclosed invention may be applied, it should be recognized that the illustrated embodiments are only preferred examples of the invention and should not be taken as limiting the scope of the invention. Rather, the scope of the invention is defined by the following claims. We therefore claim as our invention all that comes within the scope and spirit of these claims.

## Claims

1. A pharmaceutical composition for use in prevention or treatment of proliferative vitreoretinopathy (PVR), comprising an inhibitor of at least one of the activation or kinase activity of transforming growth factor β activated kinase 1 (TAK-1), wherein the inhibitor is selected from 5Z-7-oxozeaenol or an activator of protein phosphatase 6 (PP6).

2. The pharmaceutical composition for use in prevention or treatment of PVR according to claim 1, wherein the inhibitor is administered during or after surgery for treatment of retinal detachment.

## Patentansprüche

1. Eine pharmazeutische Zusammensetzung zur Verwendung bei der Prävention oder Behandlung von proliferativer Vitreoretinopathie (PVR), umfassend einen Inhibitor von mindestens der Aktivierung oder der Kinaseaktivität des transformierenden Wachstumsfaktors β aktivierte Kinase 1 (TAK-1), wobei der Inhibitor aus 5Z-7-Oxozeaenol oder einem Aktivator der Proteinphosphatase 6 (PP6) ausgewählt ist.

2. Die pharmazeutische Zusammensetzung zur Verwendung bei der Prävention oder Behandlung von PVR gemäß Anspruch 1, wobei der Inhibitor während oder nach einer Operation zur Behandlung einer Netzhautablösung verabreicht wird.

## Revendications

1. Composition pharmaceutique destinée à être utilisée dans la prévention ou le traitement de la vitréorétinopathie proliférative (VRP), comprenant un inhibiteur d'au moins une action parmi l'activation ou l'activité de kinase de la kinase 1 activée par le facteur de croissance transformant β (TAK-1), dans laquelle l'inhibiteur est sélectionné parmi le 5Z-7-oxozeaenol ou un activateur de la protéine phosphatase 6 (PP6).

2. Composition pharmaceutique destinée à être utilisée dans la prévention ou le traitement de la VRP selon la revendication 1, dans laquelle l'inhibiteur est administré au cours de ou après une chirurgie pour le traitement d'un décollement rétinien.
